(19) Europäisches Patentamt
European Patent Office
Office européen des brevets

(11) EP 2 515 097 A2

(12) **EUROPEAN PATENT APPLICATION**

(43) Date of publication:
**24.10.2012 Bulletin 2012/43**

(51) Int Cl.:
***G01N 21/25*** *(2006.01)* ***G01N 21/82*** *(2006.01)*

(21) Application number: **12163033.9**

(22) Date of filing: **07.03.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **07.03.2011 US 201113932824**

(62) Document number(s) of the earlier application(s) in accordance with Art. 76 EPC:
**12158423.9**

(71) Applicants:
- **Carroll, Wallace E.**
  **Santa Barbara, CA 93108 (US)**
- **Jackson, R. David**
  **Alexandria, IN 46001 (US)**

(72) Inventors:
- **Carroll, Wallace E.**
  **Santa Barbara, CA 93108 (US)**
- **Jackson, R. David**
  **Alexandria, IN 46001 (US)**

(74) Representative: **Beyer, Andreas**
**Bressel und Partner - Patentanwälte**
**Park Kolonnaden**
**Potsdamer Platz 10**
**10785 Berlin (DE)**

Remarks:
This application was filed on 03-04-2012 as a divisional application to the application mentioned under INID code 62.

(54) **Spectrometric device**

(57) The present invention provides improved spectrometric devices useful for measuring optical quantities of a component, including a solid state LED emitter, having at least one wavelength that is matched to the wavelength that is useful for the spectral analysis of the component of interest, a photodetection cell and an optimizing configuration and permits control of the LED emitter and the detector sensitivity to provide a range of detection for the signals corresponding to the optical density of a sample being analyzed and that affords sensitivity for a desired component of a sample by minimizing the error associated with electronic components and signals. Preferred embodiments also include configurations for determining an anticoagulant therapy value that may be used to determine treatment for a patient.

Sample Tube
LED
Emitter
Optical Path
Photovoltaic Cell
Detector
Emitter Supply
D/A Converter
Controller
A/D Converter
- Minus
+ Plus
Battery Supply
(+) D/A Converter (-)
Processor
Memory
Storage Component
Screen

FIG. 1

EP 2 515 097 A2

## Description

BACKGROUND OF THE INVENTION

Field of the Invention

The present invention relates to the field of spectroscopy, and, in particular, to a spectrometric device that incorporates a solid state light source, and more particularly, a spectrometric device that is useful for determining concentration values in blood studies.

Brief description of the related art

Spectrophotometry involves the study of electromagnetic spectra and may be carried out by determining how much light a sample absorbs. This is generally done with an instrument that passes light through the sample. At the other side of the sample, there is a detector. The light that is passed through the sample is emitted from a light source and involves photons that are sent to the sample. The sample absorbs some of the photons of light, and therefore, the light reaching the detector, is less than the total number of photons emitted from the light source. Spectroscopy may therefore be used to measure the amount of an absorbing component in a sample, since the absorption of photons may bear a relationship to the component. Spectroscopic determinations are commonly carried out by measuring the intensity of the light ($I_0$) passing through the blank. In many cases, the blank may be a solution that is identical to the sample solution except that the blank does not contain the component that absorbs light. This provides a reference, since not all of the emitted light may reach the cell, including, for example, scattered light that may not reach the cell. The reference is then compared to a reading taken of light ($I$) passing through the sample solution. From the differential between the measurements, the absorbance and/or transmittance may be determined for a sample. Transmittance is generally expressed as $(I_0)/(I)$, and absorbance is generally expressed as $A = -\log_{10} T$, where A is absorbance and T is transmittance.

A spectrophotometer may be used to measure concentrations of a component, and generally, relies on Beer's Law, which provides that a linear relationship exists between absorbance and concentration. Beer's Law is sometimes expressed as: A=ebc, where A is absorbance (no units, since $A = log_{10}\ I_0/I$), e is the molar absorptivity with units of L $mol^{-1}\ cm^{-1}$, b is the path length of the sample measured in centimeters (which may be the path length of the cuvette in which the sample is contained), and c is the concentration of the compound in solution, expressed in mol L-1. Beer's law provides a linear relationship that enables linear regression techniques to identify the concentration of components in an unknown solution, where solutions of known concentration have been used to prepare a calibration curve.

There are generally two types of spectrophotometers, one of which involves the use of a single beam emitter, and another type that involves the use of a double beam where the light intensity is compared between the two light paths, one light path passing through the test sample, and the other light path passing through a reference sample. Because of the desire to utilize the wavelength ($\lambda$) at which maximum absorbance ($\lambda_{max}$) is observed for the analyte or component being measured, the spectrophotometers are generally provided with a monochromator which generally employs a diffraction grating to produce an analytical spectrum, or a plurality of photosensors. Other spectrophotometers may use infrared, but since almost every object will emit infrared radiation as thermal radiation, particularly at wavelengths greater than about 5 $\mu$m, there are special instruments and requirements for obtaining infrared spectroscopy. One example of a dual beam spectrophotometer is discussed in US Patent 5,106,190, issued on April 21, 1992 to Toshiaki Fukuma for a "Double-Beam Spectrophotometer Using a Photodiode Detector". The 190 patent discloses a spectrophotometer having a light source and a spectroscope that separates the light of the light source depending on wavelengths, and which separates the light into a first light beam and a second light beam to pass respectively through the reference cell and a sample cell. The 190 device uses a gain setter that sets the gain of a variable amplifier depending on the reference output value from the A/D converter. US Patent 7,489,398, issued on February 10, 2009 to Takashi Otoi, discloses a "Spectrophotometer". The '398 patent discloses a device for illuminating a sample with light from a light source where the transmitted light is spectrally separated by a spectrophotometer and a group of photodetection elements arranged in a configuration in positions reached by the light of the respective wavelength components produced by this spectral separation. The '398 patent discloses that the wavelength characteristic for the sample may be obtained without the need to perform wavelength scanning.

Spectrometric devices are disclosed in US patents 5,488,474 issued on January 30, 1996 for "Hadamard Phasing in a Multisource Array" and 5,257,086 issued on October 26, 1993 for an "Optical Spectrophotometer Having a Multi-Element Light Source", both to William G. Fateley. The '474 patent discloses the use of an array of light emitting diodes (LED's) or lasers configured for activation in successive encodement patterns. The '086 patent discloses an array of light emitting diodes (LED's) configured for activation in successive Hadamard encodement patterns. The '474 patent relates to reducing centerbursts in multiplexed signals by selectively adjusting the relative phasing of the plurality of electromagnetic waves in a predetermined pattern before multiplexing.

In cases where quantification of the concentration of an analyte is relied on from the spectrophotometric information, the signal range is important. Traditional spectrophotometer devices are useful, but may be limited where there exist variations in the samples containing the component to be spectrally quantified.

A need exists to provide a spectrometric device that is capable of reducing the potential errors that may otherwise be multiplied into a reading of optical density for a sample.

SUMMARY OF THE INVENTION

The present invention relates to

1. A spectrometric device comprising:

- emitter means for producing an emission of light;
- sample holding means for holding a sample;
- detector means for detecting light;
- wherein the emitter means is situated to pass light through a sample when said sample is present in said sample holding means;
- wherein said emitter means includes an emitter and means for controlling the output of said emitter.

2. The device as mentioned in 1, wherein said means for controlling the output of said emitter comprises a digital to analog converter and a power source associated with said digital to analog converter to provide power to said emitter through said digital to analog converter.

3. The device as mentioned in 2, wherein said digital to analog converter is regulatable to regulate voltage supplied from said power source to said emitter.

4. The device as mentioned in 2, wherein said digital to analog converter is associated with computing means for processing information for communication therewith, and wherein said computing means implements instructions from software to regulate the light output of said emitter through the voltage supplied to said emitter by controlling said digital to analog converter.

5. The device as mentioned in 1, wherein said detector means comprises a photovoltaic detector.

6. The device as mentioned in 5, wherein said photovoltaic detector is associated with a detector power source, said detector power source comprising a second power source, said detector power source having a detector associated digital to analog converter that regulates the voltage that flows through the detector.

7. The device as mentioned in 6, including a detector associated analog to digital converter associated with the detector and said detector power source.

8. The device as mentioned in 7, wherein said detector is configured with said second power source to provide a response to light from said emitter by providing resistance, and wherein said detector associated analog to digital converter is connected with said detector, said second power source and said detector associated digital to analog converter for communication of said detector response to a processing component.

9. The device as mentioned in 8, wherein said processing component comprises a computing component.

10. The device as mentioned in 9, wherein said computing component comprises a computer for processing information, wherein a storage component is provided, wherein software is provided on said storage component, and wherein said computing component implements instructions from said software to process the information received from said detector associated analog to digital converter.

11. The device as mentioned in 10, further including dispensing means for dispensing a reagent.

12. The device as mentioned in 11, including first heating means for delivering heat to said dispensing means to regulate the temperature of said reagent.

13. The device as mentioned in 12, including second heating means for delivering heat to said sample to regulate the temperature of said sample.

14. The device as mentioned in 13, wherein said first heating means has a control for setting a desired maintenance temperature of said reagent, and wherein said second heating means has a control for setting a desired maintenance temperature of said sample.

15. The device as mentioned in 13, including a first temperature sensor for measuring said sample temperature and a second temperature probe for measuring said reagent temperature.

16. The device as mentioned in 15, wherein said first temperature sensor and said second temperature sensor include means for communicating with said computer to provide a signal associated with a temperature condition sensed by each respective temperature sensor.

17. The device as mentioned in 10, wherein said emitter comprises at least one LED.

18. The device as mentioned in 17, wherein said storage device includes software provided with an optimization routine that optimizes the light emitted from said emitter LED to provide the detector signal output with a desired predetermined range.

19. The device as mentioned in 18, wherein said optimized predetermined range corresponds with an optimized range for a thromboplastin reagent that reacts with fibrinogen to convert the fibrinogen to fibrin, and wherein the emitter LED is provided to emit at a wavelength that is absorbed by fibrin.

20. The device as mentioned in 19, wherein said storage component includes software with instructions for implementing the storage the signal output from the detector associated analog to digital converter.

21. The device as mentioned in 20, wherein said signal output is stored as a function of time in a database.

22. The device as mentioned in 20, wherein said software includes instructions for implementing a determination of a value determinative of clotting activity of a sample containing fibrinogen, and wherein said value is based on said database.

23. The device as mentioned in 22, wherein said software is configured with instructions to implement displaying a plot of detector output values against time, and determine points along the plot, to provide a value used for administering a treatment of a drug to patient, wherein said drug is a drug that affects clotting activity.

A detector means as mentioned in the present invention is a means comprising a detector.

The present invention provides an improved spectrometric device useful for measuring optical quantities of a component. According to a preferred embodiment, the spectrometric device includes a solid state emitter, such as, for example, a light emitting diode (LED). According to a preferred configuration, the LED includes at least one wavelength that is matched to the wavelength that is useful for the spectral analysis of the component of interest. A resistive element, such as, for example, a photodetection cell, is provided to serve as the detector. Photodetection cells, for example, may comprise a silicon electrovoltaic cell, or preferably, a selenium based cell.

It is an object of the present invention to provide a spectrometric device that affords sensitivity for a desired component of a sample by minimizing the error associated with electronic components and signals.

It is another object of the present invention to provide a spectrometric device that includes a computer for processing signals produced by the detector. Preferably, the computer includes, or is linked with, a storage component where the detector signals may be stored. An analog to digital (A/D) converter is preferably provided and, according to preferred embodiments, the signal from the detector is output to the computer through the A/D converter. In some embodiments, one or more computing components are provided and are linked for communication with the detector and/or emitter.

The spectrometric device preferably includes a variable voltage power supply, such as, for example, a battery, electric transformer, or other suitable power source for powering the emitter. The spectrometric device includes an LED arrangement that provides for the selection of different intensity levels for the LED. The LED may be controlled to provide an output that will produce a resultant signal with the detector as the photons emitted from the LED pass through a sample. Preferably the wavelength such as $\lambda_{max}$, may be emitted, and the intensity of the light output is regulated.

It is another object of the present spectrometric device to provide a signal and produce outputs that may be regulated to correspond to a particular range when different reagents are used to produce the desired component that is to be detected. For example, where a sample contains a component to be analyzed, the sample may also contain additional material, which may include a reagent. One example is where the component of interest is being generated in the sample cell while the optical activity is being measured. The present device is designed to permit reaction time analysis of a component using spectroscopy to identify the component. However, the formation of the component and the reaction forming it, in some cases, may be carried out using different regents, or may produce different by products of reaction that remain in the sample. Although the emitter may emit a wavelength specific for the component to be analyzed and detected, the variation in the environment of the sample cell may affect the amount of light that reaches the detector. The range of signals from the detector may correspond with the light received. It is an object of the present invention to provide a device that permits correlation of voltage ranges by controlling the LED emitter.

It is another object of the present invention to provide a device that permits control of the LED emitter and the detector sensitivity to provide a range of detection for the signals corresponding to the optical density of a sample being analyzed.

It is another object of the present invention to provide a spectrometric device that may be programmed to provide signal ranges that are consistent with ranges for a reaction regardless of the reagent used to produce the component being analyzed.

It is a further object of the present invention to provide a spectrometric device that may be used to carry out a blood chemistry analysis for a detectible analyte that is independent of the reagent used to generate the detectible analyte.

It is another object of the present invention to provide a spectrometric device that may be used for conducting an analysis of a body fluid, such as, for example, mammalian blood, where the sample includes a component responsible for clot formation, and where the spectrometric device is designed to produce signals corresponding to the formation of a clotting component, such as fibrin, when a clot forming reagent is introduced into the sample.

It is another object of the present invention to provide a spectrometric device that includes software that is stored on a storage component that is provided to implement instructions to collect, record and process the signals provided by the detector and other components of the detector cell circuitry, such as an associated A/D converter through which the signals may be passed, in order to provide information that may be used for the treatment of a blood disorder in a patient.

It is another object of the present invention to optimize the detector signal for a component over a range of optical activity values by optimizing the emitter output intensity and the detector response.

## BRIEF DESCRIPTION OF THE DRAWING FIGURES

Fig. 1 is a schematic illustration of a spectrometric device according to the invention.

Fig. 2 is a perspective view of an exemplary embodiment of a spectrophotometric device according to the invention, shown with a cover panel being partially removed and placed to the side of the lower portion of the device.

Fig. 3 is a plot of optical activity, in units, versus time, in seconds, for a coagulation study involving a sample containing fibrinogen and a clotting reagent that is added to the sample at time To, where the detector response is plotted over the course of the fibrinogen transformation to correspond with the optical activity of the sample at the times of the optical activity readings.

## COPYRIGHT NOTICE

© 2010 Wada, Inc. A portion of the disclosure of this patent document contains material which is subject to copyright protection. The copyright owner has no objection to the facsimile reproduction by anyone of the patent document or the patent disclosure, as it appears in the Patent and Trademark Office patent file or records, but otherwise reserves all copyright rights whatsoever. 37 CFR § 1.71(d).

## DETAILED DESCRIPTION OF THE PREFERRED EMBODIMENTS

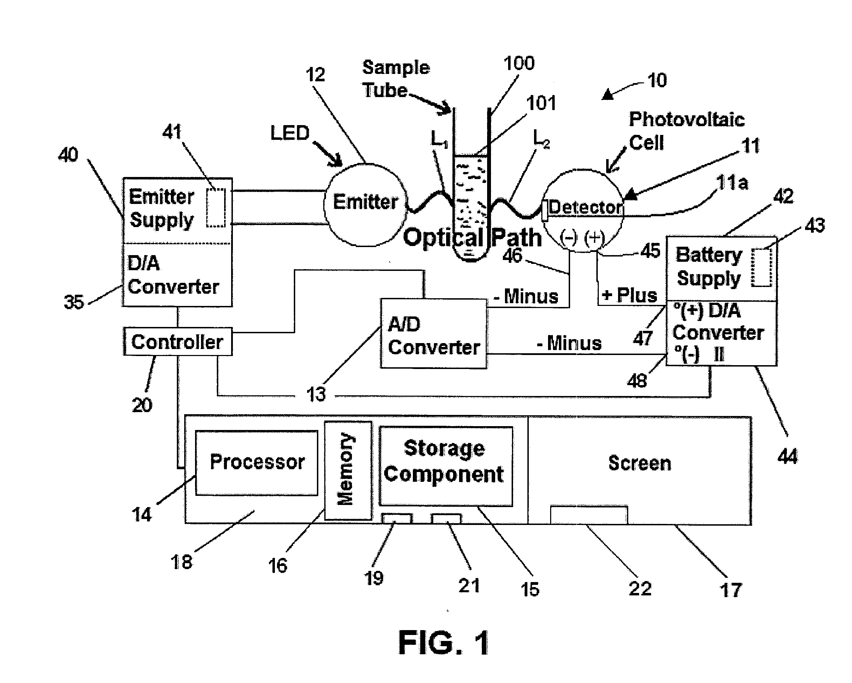

Referring to the drawings, wherein the same reference numbers indicate the same elements throughout, there is shown, in Fig. 1, a preferred embodiment of a spectrometric device 10 including a detector 11, an emitter 12, and an analog to digital (A/D) converter 13. The detector 11 may be also referred to herein as the detector cell and preferably is included in a circuit that is powered and monitored so that the voltage of the circuit that is effected by the detector 11 corresponds with the optical activity of the sample. According to a preferred embodiment, the spectrometric device 10 includes computing components, which may comprise a processor 14 and storage component 15. A memory 16, such as a random access memory, and/or an integrated memory may be provided in association with the other computing components. According to a preferred embodiment, the computing components may be associated with a board 18 that may serve to provide the electrical connections by which the computing components communicate, and which also may connect the processor 14 and/or other computing components with the other components of the device 10, such as, for example, components that may be used to supply power to or control the emitter 12 and detector 11.

The emitter 12 preferably is provided to emit light of the spectral wavelength associated with the component (the analyte) to be analyzed, and preferably emits the wavelength (λ) of which maximum absorbance ($\lambda_{max}$) is observed for the analyte. According to one preferred embodiment, the spectrometric device 10 is configured as a coagulometer and is provided with an LED emitter that outputs a suitable wavelength for the detection of an analyte that is associated with blood coagulation, such as, for example, fibrin. According to a preferred embodiment configured as a coagulometer, the LED emitter 12 is configured to emit a wavelength of 660 nm. The emitter 12 preferably is configured in a power circuit that is constructed so that the emitter 12 may be regulated to change the intensity of the LED. The LED emitter 12 preferably may be supplied with voltage that may be intermittently cycled to provide an LED emission that is less than the percentage of continuous, constant LED light. For example, the circuitry that powers the LED emitter 12 may include a clock to accomplish this.

Light emitted by the LED emitter 12 is passed through the sample container 100 and the sample 101 contained therein. An amount of light from the emitter 12 reaches the sample 101 and light is absorbed by the sample 101. An amount of light, which usually is less than what the emitter 12 emitted (e.g., due to scattering effects), is not absorbed by the sample 101 and passes through the sample container 100 and sample contents 101 (including passing through the sample container 100 to exit therefrom) to reach the detector 11. The detector 11 is a resistive element, which preferably comprises a photovoltaic cell that provides a voltage signal output based on the amount of light that it receives. According to a preferred embodiment, the resistance of the photocell detector 11 decreases as the amount of light it receives increases. In other words, the more light that the detector 11 receives from the emitter 12, the more current or voltage that is allowed to flow through the detector cell 11. The detector output preferably is passed through an analog to digital converter 13, so that the digital signal may be communicated to a component for further processing or reporting, such as, for example, the processor 14. This may be done with a controller 20 that is configured to capture the signal from the detector 11 and/or the detector associated A/D converter 13. In cases where the sample contents are changing, such as, where the sample 101 or one of its components or reaction by products undergoes a reaction, a signal sampling rate is assigned so that the computing components, such as, for example, the processor 14, storage component 15 and memory 16, directly or through the controller 20 to which the detector 11 output is provided, may obtain and preferably store signals corresponding with the optical activity of the sample at a particular time interval, such as a number N samples per rate of time, such as seconds. Preferably, the spectrometric device 10 is configured with settings that may be selectively programmed by the user, such as, for example, one-hundred samples of the photocell output signal per second are recorded. The recorded output values of the detector signal preferably are stored on a storage means such as the storage component 15. The storage component 15 may comprise a hard drive, magnetic or optical media, or other suitable storage medium. According to a preferred embodiment, the storage component 15 is provided as part of the spectrometric device 10. The spectrometric device 10 may have one or more ports 19 for accepting storage cards, such as, for example, SD, USB, and other types of flash drives or hard disk drives. In addition, the spectrometric device 10 may include a network interface card 21 or other suitable communication components that permit the spectrometric device 10 to communicate information, such as, for example, the output from the photocell detector 11, to another device, including a remote computer, terminal or portable communication device. This may be done through wired or wireless network components that are associated with the data processing components of the device 10 that process and store the sample information, such as, for example, the processor 14, storage component 15 and memory 16. Some other examples of devices to which the spectrometric device 10 may communicate information include portable or non-portable computer systems, personal digital assistants (PDAs), mobile telephones, audio/visual (A/V) devices, printers, scanners, routers, switches, gateways, firewalls, and bridges. The communication of the information from the spectrometric device 10 may be done through components or interfaces that are employed in connection with the communication of the signal from the detector 11.

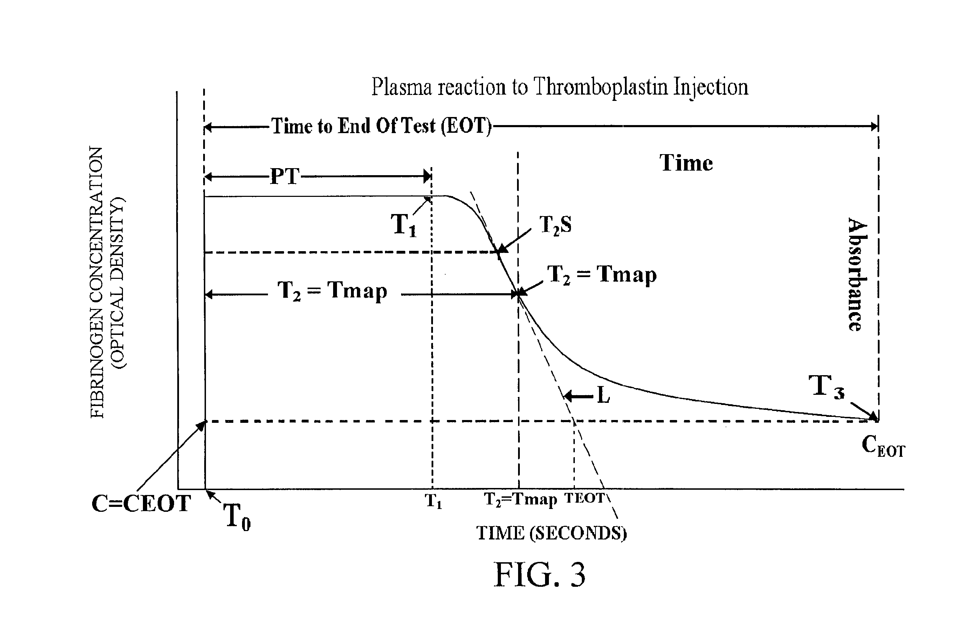

According to preferred embodiments, the processor 14 preferably may be provided with instructions to implement a determination of a value useful for determining a course of treatment for a patient. The instructions may be provided in the form of software or other removable or embedded logic, and may be stored, for example, on the storage component 15. For example, according to one preferred embodiment, the spectrometric device 10 may be used to obtain the output from the detector 11 over a period of time for a clotting reaction where a sample of human blood is reacted with a clotting reagent so that the fibrin that is produced by the transformation of the fibrinogen in the sample that is reacted with the clotting reagent may be measured as the analyte. The sampling of the detector 11 output over the course of the time of the reaction of the blood sample 101 with the clotting reagent provides optical activity values that correspond with the formation of fibrin. Preferably, the LED wavelength emitted by the emitter 12 is a wavelength that the sample analyte, here, for example, the fibrin that is produced, absorbs, and more preferably, which is the wavelength of maximum absorbance for this analyte. An example of a clotting curve is illustrated in Fig. 3, wherein optical activity values are plotted against time for a reaction involving a sample containing fibrinogen to which a reagent is added that initiates a reaction to transform the fibrinogen to fibrin. The absorbance of the emitted light by the sample 101 corresponds with the optical activity values.

According to preferred embodiments, the spectrometric device 10 may be configured to manipulate the output of the photo detector 11 to a level that corresponds with a range of current or voltage that comprises a broad range to correspond with the range of data that is useful for determinations that are made from the photocell detector 11 output. The manipulation of the output of the photo detector 11 may be accomplished by regulating the LED photons that are emitted by the emitter 12. The output signal of the detector 11 preferably is a voltage that is measured by taking into account the resistance of the photocell detector 11.

According to a preferred configuration, the device 10 includes a controller 20. The controller 20 handles the signals that are supplied to and received from the device components, such as, for example the emitter 12 and detector 11. According to one embodiment, the controller 20 preferably may be a 32-bit controller that permits assignment of a number of intensity levels for the LED emitter 12. The range of the intensity levels that the LED emitter 12 may emit, in turn, affects the current or voltage that the detector cell 11 allows to pass through based on the resistive properties of the detector 11. In this example, the controller 20 provides up to 1024 potential data points for the range or scale of values that the output of the detector 11 may distinguish. The range therefore may be maximized by regulating the LED emitter 12 so that the detectible range is within the potential data point range parameters. Preferably, the controller 20 is linked for communication with the processor 14 and other computing components, and the emitter 12 and/or the means used to supply power to the emitter 12. A system board 18 may be provided to link the controller 20 with the other computing components, such as the processor 14, storage component 15, memory 16 and the circuitry that operates the emitter 12 and detector 11. The controller 20 may be provided on or connected to the board 18. A screen 17 preferably is provided to display information about the components and the sample being analyzed, and preferably an input mechanism, such as for example a keypad 22, mouse, touch screen keyboard, is provided to an operator may input information and/or operate the device 10.

According to a preferred embodiment, where a sample 101 changes, such as, for example, by degrading, or undergoing a transformation over time, the LED emitter 12 is adjusted to be set to provide an intensity that may maximize the sampling range of values so that they are spread out over the range of the bits of data that the detector 11 has available to it. As illustrated in Fig. 1, the LED emitter 12 outputs light $L_1$ which passes through a sample tube, such as the cuvette 100, and is received by detection means which preferably includes a detector 11. The detector 11, for example, may be a silicon or selenium generating photocell (or other suitable photovoltaic cell). A first power source 40 is provided to power the LED emitter 12 and, in the exemplary embodiment of the device 10, comprises the means used to supply power to the emitter 12. The first power source 40 may be a battery 43 or may be electrical supply current that is transformed to a voltage that is desired for a range of voltages operable with the emitter 12. According to a preferred embodiment, a battery 41 may serve as the first power source 40 for the LED emitter 12 and may supply direct current (DC) voltage to a regulator means that regulates the voltage applied to the LED emitter 12. The regulator means may comprise a voltage regulating circuit or may comprise a clock that provides power to the LED over a particular frequency. Preferably, the regulator means is configured in conjunction with one or more of the computing components to provide computer regulated or computer assisted regulation of the LED supply voltage.

According to a preferred embodiment, referring to Fig. 1, a first converter that comprises a digital to analog (D/A) converter 35 is provided along with the first power source or emitter supply 40 and is associated therewith. The first D/A converter 35 preferably is configured having a variable range of voltages and is regulatable to operate over a range of voltages. According to preferred embodiments, the first D/A converter 35 may be set to a desired range of voltages (e.g., from -2.5 to 2.0 volts, or from 0 to 5 volts). This may be done manually, by the user operating the computer or computing components, such as the processor 14, storage component 15, and memory 16, where, according to preferred embodiments, the computing and regulating functions of the emitter 12 and detector 11 may be carried out with the spectrometric device 10. Preferably, the computing components are in communication with the first D/A converter 35 to regulate the voltage that the emitter 12 receives. A computer initiated routine may be provided and implemented by the computing components of the device 10. The routine may be provided as computer code in software that instructs the computing components to regulate the emitter supply voltage to provide a desired range, based on the output of the response of the detector 11, which is obtained and compared during the routine. The regulation also may take into account the sample container 100, the sample contents including the analyte being detected in order to regulate the detector 11 output to produce signals within a desired range. The routine may be configured in software which may implement programs or steps to ascertain the emitter voltage supplied and the detector voltage indicated in response thereto, and further to control the voltage supply and detection voltage range to optimize the device 10 for observing a reaction, such as, in particular, the coagulation studies carried out to determine clotting activity of a blood or blood component sample. An example of a routine that instructs the computing components to set the voltage or voltage range associated with the emitter 12 and/or the emitter light output is set forth below, and sets out a preferred example of source code for the optimization and control of the emitter LED 12 and detector 11.

A second power source 42 is arranged in a circuit with the photovoltaic cell detector 11. The second power source 42 preferably may be a battery 43 or may be electrical supply current that is transformed to a voltage that is desired for a range of voltages operable with the detector 11, including when those voltages are regulated to optimize the LED emitter 12. The second power source 42 preferably is configured to provide a range of voltages that power the detection cell circuit. According to a preferred arrangement, the second power source 42 is connected with the second D/A converter 44. The detector 11 preferably has a positive lead or positive terminal 45 and a negative lead or negative terminal 46. Preferably, as illustrated in Fig. 1, like poles of the second D/A converter 44 and power source 42 are connected. According to a preferred embodiment, the positive pole 47 of the second D/A converter 44 is connected to the positive pole of the second power source 42. The negative pole 46 of the photovoltaic cell detector 11 is connected to the power source 42 via the negative pole 48 of the second D/A converter 44, which, as illustrated, is arranged in the circuit with a detector associated A/D converter 13 where the negative pole 48 of the second D/A converter 44 associated with the second power source 42 is fed to the detector associated A/D converter 13. The negative pole 46 of the photovoltaic cell detector 11 is fed to the detector associated A/D converter 13. Fig. 1 illustrates a schematic diagram showing a preferred arrangement for the spectrometric device 10 where the emitter 12 and detector 11 are controllable through separately operable D/A converters including a first D/A converter 35 associated with the emitter 12 and a second D/A converter 44 associated with the detector 11. An A/D converter 13 is provided as part of the detector circuit, and preferably, the controller 20 is connected to the first D/A converter 35 and second D/A converter 44, and preferably also the A/D converter 13. Alternately, separately provides controllers may be used and assigned to the different components.

Preferably, the computing components receive the output from the detector 11 through the detector associated A/D converter 13. In this arrangement, the computing components may be provided with a target voltage range that the detector associated A/D converter 13 supplies via the detector cell 11 output. In order to obtain the desired range, the computer or computing components of the device 10 preferably contain or are communicatively linked with storage media that contains instructions, such as software, that implements routines to obtain voltage values of the emitter circuit and detector circuit, compare them, and, from the device components (e.g., emitter supply 40, emitter 12, detector 11, detector supply 42), regulate the voltage through the D/A converters 35, 44. The computing components with the instructions from the software compare responses from the emitter 12 and detector 11 and set voltage values to provide the desired ranges associated with the emitter 12, the detector 11 or both. A range of voltage values may be applied to the first D/A converter 35 to regulate the output of power by way of the first power source 40 and the powering of the LED of the emitter 12. The software may include instructions that instruct the processor and/or other computing components to conduct this routine by implementing a series of different voltage values to apply a different power to the emitter 12. The detector 11 may output a signal through the detector associated A/D converter 13 so that the computer or competing components are instructed to develop a reference based on the voltage supplied to the emitter 12 and the light received at the photovoltaic cell detector 11. Accordingly, a predetermined voltage value (or range of desired values) that are desired for the output of the detector 11 are provided, and the processor and computing components are instructed to set a voltage and voltage ranges for the emitter power source 40 and the detector power source 42. A procedure may be carried out to optimize the LED emitter 12 to obtain the desired signal at the detector 11 (which may be a signal within a desired voltage range for the sample analyte being spectrometrically evaluated). According to a preferred embodiment, in order to provide a voltage range that corresponds with an intensity or photon output of light emitted from the emitter 12 that produces a signal from the photovoltaic cell detector 11 within that desired voltage range, the first D/A converter 35 is adjusted to provide a corresponding emitter supply voltage from the first power source 40, the battery 41. Accordingly, the output signal at the detector 11 may be adjusted by regulating the second D/A converter 44 associated with the detector 11. In this manner, the voltage that is communicated between the detector associated A/D converter 13 and the computing components receiving the signal such as the processor 14, storage component 15 or memory 16, may be set to be within a range of desired voltages. According to one embodiment, the emitter 12 is powered to regulate its output $L_1$, and the detector cell circuit is operated by supplying power and regulating the power passing through the detector cell circuit. Preferably, the computing components, (e.g., the processor 14) are controlled with software that operates the computing components to control the emitter output and the detector cell voltage in a manner to optimize analyte detection.

Below is an example of source code that may be implemented, according to a preferred embodiment, to control the spectrometric device 10 in order to optimize the device 10 for a coagulation study. The source code preferably is provided in the form of software stored on the storage media, such as, for example, the storage component 15. The optimization of the LED emitter 12 may be accomplished through the following routine that may be implemented by software in conjunction with the associated components of the device 10, including the processor 14 and memory 16. The source code and routine below include reference to a DAQ card that may function as a controller 20 to handle the A/D conversions. According to a preferred embodiment, the card is an Advantech PCI multifunction card with A/D and D/A configuration options.

```
'Declaration definitions for SUBs that relate to the configuration

DECLARE SUB InitializeHardware ()
DECLARE SUB MonitorSystem ()
DECLARE SUB OptimizeLED ()
DECLARE SUB SetCFG (Vlu%)
DECLARE SUB SetLed (Vlu%)

DIM SHARED PARAM%(60)

 'Assign initial Advantech Parameters
 PARAM%(0) = 0                    ' Board number
 PARAM%(1) = &H300                ' Base I/O address
 PARAM%(2) = 0                    ' DMA Channel for Buffer A
 PARAM%(3) = 0                    ' DMA Channel for Buffer B
 PARAM%(4) = 5                    ' IRQ level : IRQ2
 PARAM%(5) = 100                  ' Pacer rate = 2M / (100 * 200) = 100 Hz
 PARAM%(6) = 200                  '
```

```
 PARAM%(7) = 0                  ' Trigger mode, 0 internal : 1 External
 PARAM%(8) = 1                  ' 0:Non-cyclic 1:Cyclic
 ' PARAM%(9) = RESERVED
 PARAM%(10) = VARPTR(ADA%(0))   ' Offset of A/D data buffer B
 PARAM%(11) = VARSEG(ADA%(0))   ' Segment of A/D data buffer A
 PARAM%(12) = VARPTR(ADB%(0))   ' Offset of A/D data buffer B
 PARAM%(13) = VARSEG(ADB%(0))   ' Segment of A/D data buffer B
 PARAM%(14) = 10                ' A/D conversion number
 PARAM%(15) = 12                ' A/D conversion start channel
 PARAM%(16) = 12                ' A/D conversion stop channel
 PARAM%(17) = 2                 ' Overall gain code, 0 : +/- 5V
 PARAM%(20) = VARPTR(DAA%(0))   ' Offset of D/A output data buffer A
 PARAM%(21) = VARSEG(DAA%(0))   ' Segment of D/A output data buffer A
 PARAM%(22) = 0                 ' Offset of D/A output data buffer B
 PARAM%(23) = 0                 ' Segmemt of D/A output Data buffer B
 PARAM%(24) = 1                 ' D/A conversion number
 PARAM%(25) = 0                 ' D/A conversion start channel
 PARAM%(26) = 0                 ' D/A conversion stop channel
 PARAM%(27) = VARPTR(DIA%(0))   ' Offset of D/I data buffer B
 PARAM%(28) = VARSEG(DIA%(0))   ' Segment of D/I data buffer A
 PARAM%(29) = 0                 ' Offset of D/I data buffer B
 PARAM%(30) = 0                 ' Segment of D/I data buffer B
 PARAM%(31) = 1                 ' Number of digital inputs
 PARAM%(32) = 0                 ' D/I port selection
 PARAM%(33) = VARPTR(DOA%(0))   ' Offset of D/O data buffer B
 PARAM%(34) = VARSEG(DOA%(0))   ' Segment of D/O data buffer A
 PARAM%(35) = 0                 ' Offset of D/O data buffer B
 PARAM%(36) = 0                 ' Segment of D/O data buffer B
 PARAM%(37) = 1                 ' Number of digital outputs
 PARAM%(38) = 0                 ' D/O port selection

'========================================================================
======
'POTENS+ Source PROGRAM STARTS HERE
      'Set Default values and Begin POTENS+
      Dbg = False
      LedV = 1420
      CfgV = 809

      InitializeHardware

      'Optimize the LED and get the Thromboplastin to be used
      CLS
      LOCATE 1, (79 - LEN(Msg$)) \ 2: PRINT Msg$
      OptimizeLED

'Enter the Selection menu and execute the selection
DO
      Selection = DisplayMenuMaster
      ExecuteMain (Selection)
 LOOP UNTIL Selection = 0
END
'POTENS+ Source PROGRAM ENDS HERE
'========================================================================
======
```

```
'======================================================================
======
SUB InitializeHardware

' Initialize Advandech DAQ card A/D section

      ' First Step is Initialize the Hardware Driver
      FUN% = 3    ' Func 3 : Hardware initialization
      CALL PCL812(FUN%, SEG PARAM%(0))
      IF PARAM%(45) <> 0 THEN PRINT "DRIVER INITIALIZATION FAILED !":
STOP

      FUN% = 4   ' Func 4 : A/D initialization
      CALL PCL812(FUN%, SEG PARAM%(0))
      IF PARAM%(45) <> 0 THEN PRINT "DRIVER INITIALIZATION FAILED !":
STOP

      ' Set Configuration and Led Voltage values
      FUN% = 12   ' Func 12 : D/A initialization
      CALL PCL812(FUN%, SEG PARAM%(0))
      IF PARAM%(45) <> 0 THEN PRINT "D/A INITIALIZATION FAILED !": STOP

      ' MGPL: D/A Channel 0 is CfgV on POTNES0002.  PARAM%(25) and
PARAM%(26) 1v
      SetCFG (CfgV)

      ' MGPL: D/A Channel 1 is LedV on POTNES0002.  PARAM%(25) and
PARAM%(26)
      SetLed (LedV)
      ' future addition for Heater readings improvements.
      '  BlockRead = ADA%(0)                          ' Get Reading
      '  BlockVolt = (10 * BlockRead) / 4096 + (-5)   ' Convert to
voltage
      '  BlockTemp = (BlockVolt - 1) / .04            ' Calculate
Temperature Celsus
      '  BlocTempF = (9 * BlocTempC / 5) + 32 + .05  '

END SUB


'======================================================================
======
SUB MonitorSystem

      CLS 0
      Msg$ = "Option 6 - POTENS DIAGNOSTIC"
      LOCATE 1, 1: PRINT Msg$
      LOCATE 20, 28: PRINT "Press [Esc] key to Exit"

      DO
            FUN% = 5
            CALL PCL812(FUN%, SEG PARAM%(0))
            IF PARAM%(45) <> 0 THEN PRINT "A/D SOFTWARE DATA TRANSFER
FAILED !": STOP
            Value = 0
```

```
            FOR i = 0 TO PARAM%(14) - 1
                  Value = Value + ADA%(i)
             NEXT i
            Avg = Value / PARAM%(14)
            'Display Solar Cell OUTPUT
            LOCATE 11, 6: PRINT USING "         ##.###     ####"; (Avg /
4096) * 5; Avg
            LOCATE 14, 6: PRINT USING "         ##.###     ####"; (LedV
/ 4096) * 5; LedV

            'IF LedV > 1610 THEN
            '    Blk = True
            '    LOCATE 29, 1: PRINT "Optical Path is Blocked.  Remove
Obstruction.  Press [ENTER].";
            '    WHILE INKEY$ <> CHR$(13): WEND
            ' END IF

            'IF LedV > 1800 THEN
            '    LOCATE 29, 1: PRINT SPACE$(79);
            '    Msg$ = "Check Connections to WHITE and BLACK Box or
Replace LED."
            '    LOCATE 10, (79 - LEN(Msg$)) \ 2: PRINT Msg$
            '    Msg$ = "[ Cannot CONTINUE ]"
            '    LOCATE 29, (79 - LEN(Msg$)) \ 2: PRINT Msg$
            '    END
            ' END IF
            'IF LedV > 1640 THEN
            '        Msg$ = "Optical Path Obstructed or LED needs
Replacing."
            '        LOCATE 29, 1: PRINT Msg$;
            '    ELSE
            '        LOCATE 29, 1: PRINT SPACE$(79);
            ' END IF
            'Read BlockTemp! and display
            'Read ArmTemp! and display

      LOOP UNTIL INKEY$ = CHR$(27)
      LOCATE 29, 1: PRINT SPACE$(79);

END SUB
```

**SUB OptimizeLED**

```
'
      COLOR BWHITE
      Msg$ = "Insure Optical Path is Clear.  Then Press [SPACE] to
Continue."
      LOCATE 28, (79 - LEN(Msg$)) \ 2: PRINT Msg$
      ALERT (2)
      WHILE INKEY$ <> CHR$(32): WEND
      LOCATE 28, 1: PRINT SPACE$(79);

      IF Dbg = True THEN
            Msg$ = "Optimizing Optical Path"
            LOCATE 29, 1
            PRINT Msg$;
            END IF
```

```
        DO
              FUN% = 5
              CALL PCL812(FUN%, SEG PARAM%(0))
              IF PARAM%(45) <> 0 THEN PRINT "A/D SOFTWARE DATA TRANSFER
FAILED !": STOP
                    Value = 0
                    FOR i = 0 TO PARAM%(14) - 1
                          Value = Value + ADA%(i)
                     NEXT i
                    Avg = Value \ PARAM%(14)
                    CVolt = (Avg / 4096) * 5

                    ' Adjust LedV to set value to 3770
                    IF Avg < 3769 THEN LedV = LedV + 1: SetLed (LedV)
                    IF Avg > 3771 THEN LedV = LedV - 1: SetLed (LedV)

                    'Set and an away out if Avg not needed to be met
                    'IF OLedV = LedV THEN
                    '         EXIT SUB
                    '    ELSE
                    '         OLedV = LedV
                    ' END IF

                    IF LedV > 1800 THEN
                          LOCATE 29, 1: PRINT SPACE$(79);
                          Msg$ = "Check Connections to WHITE and BLACK Box
or Replace LED."
                          LOCATE 10, (79 - LEN(Msg$)) \ 2: PRINT Msg$
                          Msg$ = "[ Cannot CONTINUE ]"
                          LOCATE 29, (79 - LEN(Msg$)) \ 2: PRINT Msg$
                          END
                     END IF
       LOOP UNTIL INKEY$ = CHR$(27) OR Avg > 3768 AND Avg < 3772

 LOCATE 29, 1
 PRINT SPACE$(79);
END SUB


SUB SetCFG (Vlu%)
'
      DAA%(0) = Vlu%                ' Set Configuration output Voltage
(819)
      PARAM%(25) = 0                ' D/A conversion start channel
      PARAM%(26) = 0                ' D/A conversion stop channel
      CfgV = DAA%(0)
      FUN% = 13   ' Func 13 : "N" times of D/A output
      CALL PCL812(FUN%, SEG PARAM%(0))
      IF PARAM%(45) <> 0 THEN PRINT "D/A OUTPUT FAILED !": STOP
END SUB

'Set LED by changing the supply voltage the reading the CELL output.
SUB SetLed (Vlu%)
'
      DAA%(0) = Vlu%                ' Set LED output Voltage (1507)
1.81v
```

```
        PARAM%(25) = 1                ' D/A conversion start channel
        PARAM%(26) = 1                ' D/A conversion stop channel
        LedV = DAA%(0)
  '
        FUN% = 13   ' Func 13 : "N" times of D/A output
        CALL PCL812(FUN%, SEG PARAM%(0))
        IF PARAM%(45) <> 0 THEN PRINT "D/A OUTPUT FAILED !": STOP
  '
  END SUB
```

The above routines may be implemented in whole or part to provide optimization of the LED emitter 12. Alternatively, other optimization routines may be implemented to regulate the LED emitter 12 and the detection cell voltage.

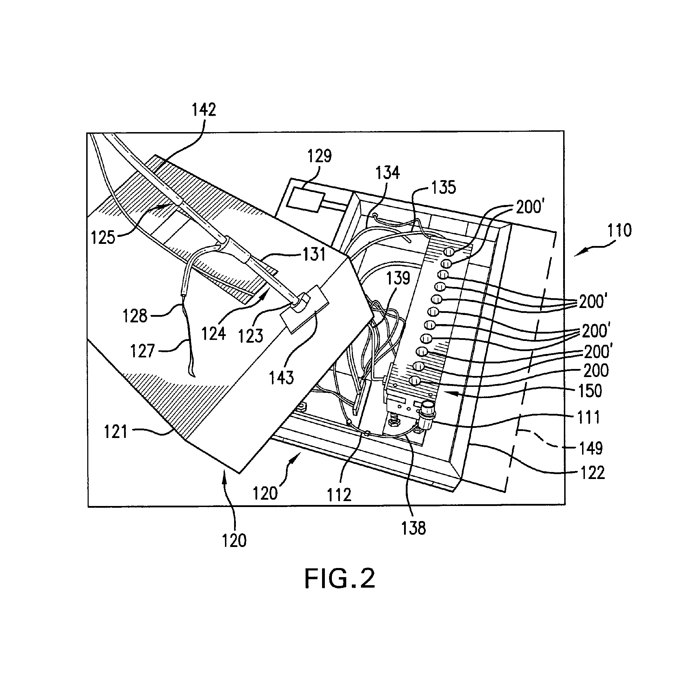

Referring to Fig. 2, an alternate embodiment of a spectrometric device 110 is illustrated including an emitter 112 and a detector 111. The device 110 may be operated and controlled similar to the device 10 shown and described herein. The spectrometric device 110 is illustrated with a sample holding mechanism and reagent delivery mechanism. A device housing 120 is provided to hold the components of the device 110, and, preferably, may include an upper or first housing part 121 and a lower or second housing part 122 that may be arranged together to form the housing 120. The housing parts 121, 122 may be removably detachable to provide access to the components enclosed therein. A reagent holding mechanism 125 is shown with a holder 123 for holding the reagent arm 124 provided on the upper housing part 121. The housing 120 may be constructed from aluminum, plastic, including molded plastic, stainless steel, or other suitable compositions, and, preferably, may be insulated to retain the temperature of the sample or samples placed therein. The emitter 112 is shown positioned on one side of the sample slot 200 and the detector 111 is provided with a photovoltaic detector cell positioned opposite of the emitter 112 on the other side of the sample slot 200. A heating block 150 is provided and contains a heating mechanism to provide a temperature controlled environment for the sample 101 (and other samples that may be held in the block 150). Preferably, the sample slot 200 is formed in the heating block 150. A plurality of holding ports 200' for holding sample cuvettes is provided in the heating block 150. Accordingly, a sample cuvette, such as that 100 shown in Fig. 1, may be placed in the sample slot 200 or in one of the holding ports 200' in order to reach or maintain a desired temperature for testing. A number of sample cuvettes may be held in the block 150 until they are to be spectrometrically read (and, in the case of the coagulation study, until they are to be spectrometrically read and reacted with a thromboplastin or clotting agent added thereto).

The regent holding mechanism 125 is also shown with the reagent arm 124 seated in the holder 123 attached to the upper housing 121. The wires 127, 128 wrapped by the shrink tubing 130 are for a heater and sensor combination which, although not shown, are disposed in the reagent arm sleeve 131. The sleeve 131, for example, may comprise a copper tube or other suitable insulating component. A reagent heating mechanism includes the heater in the sleeve 131 and controls the temperature of the reagent prior to injection into a sample container 100. Although the heater is preferably located in the arm sleeve 131, the reagent may be preheated at its source or at a location prior to the reagent passage through the reagent arm sleeve 131. The reagent holding mechanism 125 holds the reagent at a desired temperature so that it is available for dispensing into a cuvette 100 containing a sample to be evaluated. The reagent holding mechanism 125 preferably includes a supply line 142 through which reagent may be delivered and from which the reagent may be dispensed. A reagent source, such as, for example a reservoir (not shown) may be provided in communication with the reagent supply line 142 so that the reagent supply line 142 may pass the reagent through to the sample holder, such as a cuvette 101. The reagent holding mechanism 125 may be sized to accommodate the amount of reagent that is required for a particular sample run (e.g., a single sample versus a number of samples), and the dimensions and size of the supply line 142 and reservoir (not shown) may be constructed to have suitable size dimensions for the amount of reagent that is required. The reagent holder heating mechanism preferably is configured so that the supply line 142 passes through the sleeve 131. Preferably, the sleeve 131 is provided proximal to the dispensing end 143 of the supply line 142. The sleeve 131 may be constructed from a material that suitably maintains the temperature of the reagent, and allows the heating mechanism, such as, for example, copper. The heating element (not shown) and a sensor (not shown) preferably are provided within the sleeve 131 to maintain the temperature of the reagent present in the supply line 142, or that passes through the portion of the supply line 142 disposed within the sleeve 131.

Alternately, though not shown, the sleeve 131 may be extended to cover additional portions or lengths of the supply line 142. The heating element (not shown) may be operated with a power supply fed through the wires 127, 128. Wires 127, 128 also may transmit the signal from the sleeve sensor (not shown) to an analog to digital A/D converter to provide a temperature reading. The reagent holder heating mechanism may include an associated A/D converter (not shown) and communicate its output to the computer component or computer. Preferably, the A/D converter associated with the reagent arm temperature sensor is linked with the computer or the computing component of the device 110 to transmit the sensor output so the computer or computing components which preferably include software with instructions, may read and store the information in accordance with the instructions of the software implemented by the computer or computing components. According to a preferred embodiment, software may be provided that is configured with instructions for recording the temperature readings of the sensor and for controlling the heater to maintain a desired reagent temperature with the sleeve 131 for delivery to a sample.

Though not shown, a pump may be provided to supply reagent through the reagent supply line 142 and administer injection of the reagent into the sample container 100. The pump may include circuitry that is operable through software, and the computer or the computing components may manage the operation of the pump to control the injection of reagent, which may include controlling one or more of the volume of reagent, the time of injection of the reagent, and the temperature of the reagent.

As shown in Fig. 2, the heating block 150 is configured with a main sample slot 200 and a number of additional sample slots or holding ports 200'. The sample slot 200 is within the optical path, with the LED emitter 112 shown on the left and the photo cell detector 111 shown on the right. The detector A/D values are read from the controller 129 or data acquisition card (e.g., an Advantech multifunction DAQ PCI card). The arrangement of the detector circuit and the emitter circuit in the device 110 may be configured similar to the circuits shown and described in connection with the device 10 of Fig. 1. The card or controller 129 preferably monitors and with the other computing components, such as, for example, those shown and described in connection with the device 10 of Fig. 1, records the detector signal passed through the wire leads 138 that connect with the detector 111 and/or detector circuitry. The D/A voltage is sent to the LED emitter 112 from the DAQ card or controller 129 through the wire leads 139 which may connect with the components of the device 110 that receive and effect the voltage signals regarding the detector 111 and emitter 112. Wires 134, 135 enter the back of the aluminum heating block 150 and attach to header/sensor combination for the heating block 150 similar to the components discussed and shown in connection with the reagent arm 124 and the wires 127, 128 that are shown for the reagent arm heater and sensor housed in the arm sleeve 131. According to one embodiment, the initial settings of the heater associated with the reagent heating arm 124 that is in the sleeve 131 and the heater of the heating block 150 preferably are done manually and may be done using a calibrated thermometer and a small tool, such as a screwdriver. Alternately, a temperature control unit or computer implemented control may be used to control and operate the heating and temperature sensing functions of the heating block 150 and of the reagent heating mechanism of the heating arm 124 and sleeve 131. According to one embodiment, the heating block 150 and arm 124 may have independent heating controls. Alternatively, the heating mechanism of the arm 124 and heating block 150 may be preset to a desired temperature for a coagulation study reaction involving fibrinogen, or the device 110 may include an option for selecting the coagulation study where the heating temperatures may be preset to a desired temperature or temperature parameters for a clotting reaction.

According to preferred embodiment, the sample heating mechanism, such as, for example, the heating block 150, is designed to impart heating of the sample contents 101 in order to raise the sample temperature and, preferably, to maintain the sample temperature at a desired level, or, where a preheated sample is involved, to continue to maintain the sample temperature. Preferably, the heating block 150 is constructed from a material that may be elevated to a desired temperature and which preferably is able to maintain the desired temperature. One preferred material for the construction of the heating block 150 is aluminum. The heating block 150 preferably is connected to a supply for powering the heating block 150 heater mechanism, and is provided with a temperature sensor (not shown) whose output is transmitted through an A/D converter (e.g., through the wire leads 139). The heating block A/D converter preferably communicates the output to the computing components (such as those including the processor 14, storage component 15 and memory 16 described in connection with the device 10), and the signal values corresponding with the heating block 150 temperatures may be stored or reported, including being made instantaneously available for reading or viewing on a screen or monitor 149 which may be provided on or in connection with the device 110. Although the screen 149 is shown on the right side of the device 110 in Fig. 2, the screen 149 may be provided on another location of the device 110. Although not shown, the device 110 may be configured to include some of all of the components described in connection with the device 10 of Fig. 1, such as, for example, the input mechanism, board, network interface, storage component, memory, and processor. The computing components preferably are provided with software that is stored on a storage component of the device 110 and implements monitoring of the heater block temperatures and includes instructions for operating and controlling the heating mechanism of the heating block 150 in order to maintain a constant or desired sample temperature. According to a preferred embodiment, the spectrometric device 110 may be used to carry out evaluations and analyses of reactions taking place in the sample container 100.

A preferred operation of the spectrometric devices 10, 110 according to the invention is described in connection with the exemplary diagram of the device 10 shown in Fig. 1 and the exemplary embodiment shown and described in relating to Fig. 2. Although discussed in connection with the device 10, the operation of the spectrometric device 110 may be carried out in a similar manner. The spectrometric device 10 is designed to supply current to the emitter 12 and to the detector 11, which may be accomplished with two separate circuits. The output voltage of the emitter associated first D/A converter 35 is controllable, and, for example, may be controlled by a 12 bit word where a bit value = 0.0048876 volts (e.g., 5 volts / 1023 steps). The 1023 steps in the preferred embodiment illustrated herein, provide for a range of 1023 steps. For example, the voltage range of the A/D and D/A converters may be made variable by a computer initialization routine. For an example: (0 to 5 volts) or (-2.5 to + 2.5) volts may be set. According to one example, the computer or computing components are operated with software that contains instructions for optimizing the LED emitter output through implementation of a routine that may evaluate the LED light output and detector 11 response at various step levels. Once a step level is associated with conforming to an optimized LED output, then steps around that level may be evaluated to fine tune the optimization of the LED output. According to a preferred configuration, when a sample 100 is placed in the detection path (e.g., between the emitter 12 or 112 and the detector 11 or 111) the computer or computing components are programmed to store the converter A/D data from the detector circuit that corresponds with the output of the detector 11, 111. Where the emitter 12, 112 produces a wavelength to which the analyte being measured responds (e.g., through absorbance) and some light is passed through the sample and sample container to the detector 11, 111. The detector data derived from the detector response (e.g., a signal) to the light received through the sample path when a sample is present in the light path, corresponds with the concentration of the analyte in the sample 101 at the time of measurement. Preferably, the data obtained by the device components is stored in an array for processing. The data may be obtained and stored to represent a data point at a particular time interval (e.g., 1/100th of a second). The processes may be configured to display the curve and compute various points on the curve to determine results, as shown by the clotting curve represented in Fig. 3, which shows a spike in the optical activity upon the reagent addition and follows the transformation reaction over time, until the reaction subsides.

The data preferably is obtained by the detector 11 and its corresponding signal output which the computing components in connection with the detector circuitry may monitor and store. The detector 11 preferably includes a photovoltaic cell and a second power source 42 that powers the detector circuit. The second power source 42 preferably may comprise a constant voltage DC source, and is provided in a circuit with the photovoltaic cell 11 a of the detector 11. According to a preferred arrangement, the voltage of the constant voltage DC source is changed as a result of the resistance of the photocell detector 11 based on the light $L_2$ (see Fig. 1) received by the photocell detector 11. Preferably, a reference voltage or reading is obtained based on the photocell detector reaction to the emitter 12 providing light when no sample is contained in the light path. The reference voltage may be obtained by leaving the sample path clear of a sample and a sample holder, or, alternately, may be obtained by placing the sample holder in the path empty or with water (or other solvent). In some instances, the solvent that the sample analyte is contained in may be used to obtain the reference voltage. Because other processes may reduce the intensity of the light that passes through the cuvette, in some cases, a background reading is taken for the solvent and the cuvette or sample holder.

The voltage output that the detector 11 provides based on the LED emitter light that the detector 11 receives, preferably is passed through the A/D converter 13, and a digital voltage value is provided that corresponds with an amount of light passing through the sample, and bears a relationship to the component or analyte in the sample based on the light absorbed by the analyte. The voltage values from the detector 11 may be processed with the computing components, such as, for example, the processor 14, storage component 15 and memory 16 of the device 10, and software that is configured with instructions to implement the functions of the processes. The device 10 may be programmed to relate the voltage values to absorbance based on the relative values of the voltage values obtained and measured with the detector cell 11a and A/D converter 13 of the detector circuit.

One example of a relationship between absorbance of the sample at a particular time and the voltage detected at that time is expressed by the following equation (III):

$$A = -\log_{10}((V_{\text{sample}} - V_{\text{zero}})/(V_{\text{zero}})) = \varepsilon cl \qquad \text{(III)}$$

A represents absorbance, $V_{zero}$ represents the value of the voltage when the light of the emitter 12 reaches the detector 11 with no sample holder or solvent in the light path. In embodiments where the spectrometric device 10 is configured to record a reference for the sample holder 100 and solvent that are placed in the light path for the zero reference voltage, then the denominator is adjusted by subtracting from the value $V_{zero}$ the value of the voltage with the sample holder and solvent in the light path so that the denominator of expression (III) above, is ($V_{\text{solvent+ sample holder}} - V_{zero}$)

According to a preferred embodiment, where the spectrometric device 10 is utilized in connection with a sample containing an analyte that undergoes a reaction change or where the analyte itself is a result of a reaction (e.g., it is produced by the reaction or is a by product of the reaction), then the spectrometric device 10 may be configured with a reagent delivery system, including, for example, a reagent holding arm 131, as shown in Fig. 2 in connection with the device 110. One or more temperature control mechanisms may be provided to maintain the reagent at a desired temperature, including those discussed herein. For example, according to preferred embodiments, the sample may be held in a sample holder that includes a heating mechanism to facilitate maintenance of the sample at a desired temperature (see e.g., the heating block 150 of Fig. 2).

For example, preferably, the detector cell 11 is positioned adjacent an opposite wall of the sample container 100, and the emitter light source 12 positioned adjacent an opposite wall, so the light $L_1$ emitted from the emitter light source 12 passes through the container 100. The emitter light source 12 is preferably configured to produce light $L_1$ which can be absorbed by one or more components in the sample 101 which are to be measured, or one or more components that may be generated in the sample as a result of a reaction taking place. Preferably, the devices 10, 110 may be used to detect a single component that has maximum absorbance at a particular wavelength.

The spectrometric devices 10, 110 may be used to carry out coagulation studies, including those discussed in my US patent nos. 6,706,536 and 7,276,377 which involve, *inter alia,* determinations of anticoagulant therapy factors for patients with blood disorders. In those patents, reference is made to my prior patent 5,502,651, where a potentiophotometer is used to determine optical density values for a sample containing fibrinogen, and the values of the sample when the fibrinogen undergoes a transformation to fibrin, upon a reagent addition to the sample. Devices 10, 110 according to the present invention may be used to define the range of detection for an analyte determination in a way that broadens the range to increase the sensitivity and the detection of changes. One particular use of the devices 10, 110, is where the device is configured as a blood coagulant determination device, with the computing components being programmed to regulate the emitter 12 output, the detector 11 circuit, and store and process the stored information to derive an anticoagulant therapy factor for a blood sample.

In accordance with a preferred embodiment of the present invention, the emitter 12, for example, may include one or more a light emitting diodes (LEDs) emitting a predetermined wavelength, such as for example, a wavelength of 660 nm, and the detector cell 11 may, for example, comprise a silicon photovoltaic cell detector. Optionally, though not shown, a bar code reader may also be provided to read bar code labels placed on the sample container 100 (at a location that does not interfere with the light path of the emitter 12, 112). The bar code reader may produce a readable, unique signal or code which can be read by the computer or computing components to associate a set of data with a particular sample container 100. The bar code reader may be connected to the board 18 or a controller, such as for example, the controller 20.

The spectrometric device 10 may be configured with software which preferably is stored on a storage component 15 of the device 10, such as, for example, storage media, (e.g., a hard drive). The device 10 may be configured to obtain coagulation analysis information, and the information obtained may then be used to determine the treatment that is needed for an individual (such as, for example, blood thinners or the like). The sample 101, preferably, for this example, is a blood sample or blood component sample. The device 10 is operated by taking readings of the optical activity of the sample 101 before, during and after a clotting reagent is added to the blood or blood component sample 101. The reagent is a clotting reagent that will cause the fibrinogen transformation to fibrin, so that fibrinogen in the sample will undergo a transformation to fibrin. The emitter 11 is preferably configured to produce an optical wavelength that corresponds with the maximum wavelength $\lambda_{max}$ for the fibrin, which is about 660 nm. The emitter 11 produces the wavelength, and preferably, is adjusted through the operation of an optimization routine, such as the optimization routines, discussed herein, to optimize the emitter 12 output and detector 11. The device 10 preferably is instructed to operate the emitter 12 to emit the optimized output at the desired wavelength, which may be accomplished with software provided on the device 10, 110. The device 10 also preferably may include a reagent injection element, such as, for example, the reagent arm 124 that maintains the reagent at the desired temperature for addition to the sample 101. The device 10 though not shown, also may include a manual reagent arm 124 that draws reagent from a reservoir (not shown) that is maintained at a desired temperature. The reservoir may have a line 142 coming from it that delivers the reagent to the dispensing tip 143. A pump or other device may be used to deposit the reagent amount into the sample. According to one embodiment, the pump may be configured with a timer to operate for a predetermined amount of time to correspond with the delivery of the reagent amount desired to be injected or otherwise delivered to the sample 101. The pump (not shown) may be controlled by a computer, such as the computing components of the device 10, and may correspond with the detector readings, such as, for example, timing an addition of reagent to the sample to take place after one or more initial readings of the detector signal output has been recorded, and recording the time of the addition of the reagent.

Preferably, the software is configured to implement detection of optical activity by having the detector signal read every n number of times per unit of time, such as a number of times per second. The optical activity corresponding to the sample, in this case, the formation of fibrin from the fibrinogen in the sample 101 as a result of the reagent addition to the sample 101 is recorded and the time interval is recorded so that there is an array of data that includes optical activity values at corresponding time values. The plot of optical activity against time results in the clotting curve similar to that illustrated in Fig. 3.

Preferably, the data is stored on the storage component 15, and the data is used so that an algorithm may be applied to manipulate the data using the processor 14 of the device 10 to provide a resultant coagulation value, which, preferably may be an International Normalized Ratio (INR) for a sample 101 such as a blood sample or blood component sample. The determination of an INR value may enable treatment to be administered to an individual since it provides a measure corresponding with the individual's clotting activity, based on the sample evaluated with the coagulation reaction and the optical activity detected.

The device 10 may be configured to implement reporting of the clotting activity data. According to one embodiment, the device 10 is configured to identify a sample, by operator input, or by a bar code assigned to or associated with the sample 101, or other unique identification mechanism. The sample identification and the corresponding data for the sample are stored as a sample record. The INR for the sample record may be reported. The data from which the INR is determined also may be reported as desired, in the event that it is desirable or necessary to view or access it. The present device 10 enables a number of samples to be processed and have their optical activity determined for a clotting reaction, and the information corresponding to each sample's clotting activity may be generated in a report form (including with such information as, sample ID, date/time that the sample was taken and INR), date of the test. Additional parameters that the report may include may be any of those observed, set or processed, such as, device settings (voltages of the emitter 12, detector 11, as well as the name of clotting reagent used, its manufacturer, international sensitivity index (ISI) and batch number).

The device 10 preferably may include software that is programmed to implement a comparison routine that correlates different reagents to conform to a single reagent parameter. For example, according to a preferred embodiment, the thromboplastin reagent Thromboplastin C (from a particular manufacturer) may be set to be the standard, and the device 10 may be provided with software that is configured to relate the other clotting reagents, such as, for example other thromboplastins (from other manufacturers), Innovin, BPT or other clotting reagents. An algorithm may be stored in the software and implemented to process data. The device 10 preferably includes an input means, such as, for example, a keyboard or key panel, and may include a mouse or other selection device, that permits a user to enter information corresponding with a reagent, such as the reagent name, manufacturer, date, and international sensitivity index (ISI) for that reagent (which is a value that the manufacturer assigns to that lot of reagent). The correlation algorithm preferably relates the coagulation data, such as, for example, the INR for the sample, to correspond with a single reagent, such as, for example Thromboplastin C (TPC), even where other clotting reagents may be used to carry out the optical value coagulation study with the device 10. The correlation of INR values obtained using the device 10 provides a way to standardize potential treatment options based on the values obtained regardless of which thromboplastin or clotting reagent is used to generate the data or INR. According to a preferred embodiment, the device 10 is configured with software that implements instructions to store and process data, and includes processing the data to determine an INR for a sample based on the following formula: $INR_{INSTRUMENT} = (T1*TEOT)$, where T1 is the time measured, after the injection or addition of the clotting agent, at which the fibrinogen in the sample begins to transform to fibrin, and where TEOT is a theoretical end of the test. The program may be configured to determine the TEOT with a theoretical or hypothetical zero order kinetic line, or line L, as it is referred to that appears on the Fig. 3, which provides an (x,y) coordinate of (TEOT, 0). The slope of the line L may be determined based on a slope or line taken between the point where the maximum acceleration of the conversion rate of fibrinogen transformation begins (shown as T2S in Fig. 3) and the point marking the end of the maximum conversion rate (which is the last highest delta value of conversion rate), which is T2 or Tmap in Fig. 3. The value, TEOT, is a time value, and, generally, for example, may be expressed in seconds. The devices 10, 110 may be programmed to determine T1 and TEOT, including the commencement of the maximum conversion rate, and the last conversion rate value. In order to determine the TEOT, preferably, the slope of maximum acceleration on the clotting curve that corresponds with the maximum rate of formation of fibrin during the reaction of the fibrinogen in the sample with a clotting agent is determined. One preferred method for determining the slope is by determining the maximum acceleration point (identified as T2 or Tmap on Fig. 3). The determination of Tmap (also T2) may be done by instructing the processor 14 to sample and record optical activity values (e.g., such as absorbance values and their corresponding time values) of the sample and clotting agent as the clotting reaction takes place, and from this data, using the detector responses corresponding thereto (i.e., the optical activity values), having the processor 14 determine the last highest delta of an increasing rate of fibrinogen transformation. The slope is illustrated represented by the line L in Fig. 3, and the line L intersects c=Ceot, a point where the fibrinogen to fibrin transformation for the sample being analyzed has tapered off, which provides a value for the TEOT (at the intersection of L with c=Ceot.

This INR determination is further discussed in our copending patent application US Application Serial No. 12/932,822, filed on March 7, 2011, the complete disclosure of which is herein incorporated by reference. The devices 10, 110 shown and described herein may be programmed with software to implement the determinations of INR values for blood or blood components in connection with the method

The devices 10, 110 shown and described herein may be used to carry out coagulation studies for a plurality of patient records, store and communicate data to an operator of the device 10, 110, or to an alternate or remote location. In addition, although the means to supply power is illustrated as a separately provided first power source 40 and a second power source 42, according to alternate embodiments, power may be supplied through the computing components, and may be regulated with D/A converters as described to control the voltage.

According to another alternate embodiment, the device is configured to interface or connect with a computer. Software preferably is provided and is installed on the computer so that the computer may communicate with the device and control the operations of the device, as discussed herein. According to this embodiment, the device is configured with an interface, such as a port or card, that connects with a port of the computer (e.g., serial port, USB port, or other suitable port connection). The device preferably has its own power supply for supplying power to the components of the device, and the computer, configured with software, may be operated to control the emitter output, detector response, sampling times, heating block temperature and other components of the device. Preferably, the software instructs the computer to carry out monitoring functions and collects and stores data, and manipulates the data in accordance with expressions that are used to carry out a coagulation study, such as, for example, a study of a patient blood sample that undergoes a reaction with a clotting reagent (e.g., thromboplastin) to determine clotting activity based on the transformation of fibrinogen in the sample to fibrin. The spectrometric device may be constructed for carrying out the determinations set forth in our copending patent application serial no. 12/932,822, filed on March 7, 2011.

According to an alternate embodiment, the processor 14 and storage component 15 may be provided in the form of a computer (such as, for example an IBM compatible computer, or other type of computer), and linked for communication with the detector 11 and its output, and/or other component with which the detector 11 interfaces or is associated. The computer (not shown) may be linked for communication with the emitter 12 and/or other component associated with or interfacing with the emitter 12.

While the invention has been described with reference to specific embodiments, the descriptions are illustrative and are not to be construed as limiting the scope of the invention. Although the uses of the devices 10 and 110 are, at times, separately referred to in connection with a description of the methods or operations thereof, the features of the devices 10, 110 may be interchanged. For example, the reagent arm 125 and heating block 150, may be utilized in connection with the device 10, and, likewise, the components, including converters, power supplies, of the device 10 may be used with the device 110. The sample container used to contain the sample may comprise a vial, or cuvette, including, for example, the sample container disclosed in our U.S. Patent 6,706,536. For example, the spectrometric device 10, 110, although described as having use in connection with body fluids of a human, may be used in connection with veterinary procedures, as well, where fluids are to be measured or analyzed. Various modifications and changes may occur to those skilled in the art without departing from the spirit and scope of the invention described herein and as defined by the appended claims.

## Claims

**1.** A spectrometric device comprising:

- emitter means for producing an emission of light;
- sample holding means for holding a sample;
- detector means for detecting light;
- wherein the emitter means is situated to pass light through a sample when said sample is present in said sample holding means;
- wherein said emitter means includes an emitter and means for controlling the output of said emitter;

wherein said means for controlling the output of said emitter comprises a digital to analog converter and a power source associated with said digital to analog converter to provide power to said emitter through said digital to analog converter;
wherein said digital to analog converter is regulatable to regulate voltage supplied from said power source to said emitter; and
wherein said digital to analog converter is associated with computing means for processing information for communication therewith, and wherein said computing means implements instructions from software to regulate the light output of said emitter through the voltage supplied to said emitter by controlling said digital to analog converter.

**2.** The device of claim 1, wherein said detector means comprises a photovoltaic detector.

**3.** The device of any one of claims 1-2, wherein said detector means comprising a photovoltaic detector is associated with a detector power source, said detector power source comprising a second power source, said detector power source having a detector associated digital to analog converter that regulates the voltage that flows through the detector.

**4.** The device of any one of claims 1-3 , including a detector-associated analog to digital converter associated with the detector and said detector power source.

**5.** The device of any one of claims 1-4 , wherein said detector is configured with said second power source to provide a response to light from said emitter by providing resistance, and wherein said detector-associated analog to digital converter is connected with said detector, said second power source and said detector-associated digital to analog converter for communication of said detector response to a processing component.

**6.** The device of any one of claims 1-5, wherein said processing component comprises a computing component.

**7.** The device of any one of claims 1-6, wherein said computing component comprises a computer for processing information, wherein a storage component is provided, wherein software is provided on said storage component, and wherein said computing component implements instructions from said software to process the information received from said detector associated analog to digital converter.

**8.** The device of any one of claims 1-7, further including dispensing means for dispensing a reagent.

**9.** The device of any one of claims 1-8, including first heating means for delivering heat to said dispensing means to regulate the temperature of said reagent.

**10.** The device of any one of claims 1-9, including second heating means for delivering heat to said sample to regulate the temperature of said sample, wherein said first heating means has a control for setting a desired maintenance temperature of said reagent, and wherein said second heating means has a control for setting a desired maintenance temperature of said sample, and including a first temperature sensor for measuring said sample temperature and a second temperature probe for measuring said reagent temperature.

**11.** The device of any one of claims 1-10 , including means for communicating with said computer to provide a signal associated with a temperature condition sensed by each respective temperature sensor.

**12.** The device of any one of claims 1-11, wherein said emitter comprises at least one LED.

**13.** The device of any one of claims 1-12, including software provided with an optimization routine that optimizes the light emitted from said emitter LED to provide the detector signal output with a desired predetermined range.

**14.** The device of any one of claims 1-12, including software provided with an optimization routine that optimizes the light emitted from said emitter LED to provide the detector signal output with a desired predetermined range, wherein said optimized predetermined range corresponds with an optimized range for a thromboplastin reagent that reacts with fibrinogen to convert the fibrinogen to fibrin, and wherein the emitter LED is provided to emit at a wavelength that is absorbed by fibrin.

**15.** The device of any one of claims 1-12, including software provided with an optimization routine that optimizes the light emitted from said emitter LED to provide the detector signal output with a desired predetermined range, wherein said optimized predetermined range corresponds with an optimized range for a thromboplastin reagent that reacts with fibrinogen to convert the fibrinogen to fibrin, and wherein the emitter LED is provided to emit at a wavelength that is absorbed by fibrin, wherein said storage component includes software with instructions for implementing the storage the signal output from the detector associated analog to digital converter;
wherein said signal output is stored as a function of time in a database;
wherein said software includes instructions for implementing a determination of a value determinative of clotting activity of a sample containing fibrinogen, and wherein said value is based on said database; and
wherein said software is configured with instructions to implement displaying a plot of detector output values against time, and determine points along the plot, to provide a value used for administering a treatment of a drug to patient, wherein said drug is a drug that affects clotting activity.

10
Photovoltaic Cell
11
11a
Detector
(-) (+)
45
46
47
48
42
43
Battery Supply
°(+) D/A Converter °(-) II
44
+ Plus
- Minus
- Minus
100
101
$L_2$
$L_1$
Sample Tube
Optical Path
Emitter
12
LED
A/D Converter
13
Emitter Supply
D/A Converter
41
40
35
Controller
20
Screen
17
22
Storage Component
15
21
19
Memory
16
Processor
14
18


**FIG. 1**

142
129
134
135
200'
110
125
131
139
200'
128
200'
124
123
200'
127
143
200
150
111
121
149
122
120
112
138
120

FIG.2

Plasma reaction to Thromboplastin Injection
Time to End Of Test (EOT)
PT
$T_2$ = Tmap
Absorbance
Time
$T_1$
$T_2S$
$T_2$ = Tmap
L
$T_3$
$C_{EOT}$
$T_0$
C=CEOT
$T_1$
$T_2$=Tmap TEOT
TIME (SECONDS)
FIBRINOGEN CONCENTRATION
(OPTICAL DENSITY)

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5106190 A, Toshiaki Fukuma
- US 7489398 B, Takashi Otoi
- US 5488474 A
- US 5257086 A
- US 6706536 B
- US 7276377 B
- US 5502651 A
- US 93282211 A
- US 12932822 B